# EUROPEAN PATENT SPECIFICATION

(11) **EP 1 179 592 B1**
(45) Date of publication and mention of the grant of the patent: **01.02.2006**
(21) Application number: 01912238.1
(22) Date of filing: 09.03.2001
(51) Int. Cl.: C12N 15/09, C07K 14/78, C07K 14/705

(54) **PROTEINS NECTIN-3**
NECTIN-3 PROTEINE
PROTEINES DU TYPE NECTINE 3

(30) Priority: 09.03.2000 JP 2000006595
(43) Date of publication of application: 13.02.2002
(73) Proprietor: Japan Science and Technology Agency, Kawaguchi-shi, Saitama 332-0012 (JP); JCR PHARMACEUTICALS Co., LTD., Ashiya-shi, Hyogo (JP)
(72) Inventor: TAKAHASHI, Kenichi, Kobe-shi, Hyogo 655-0854 (JP); TAKAI, Yoshimi, Kobe-shi, Hyogo 651-2102 (JP); NAKANISHI, Hiroyuki, Kobe-shi, Hyogo 651-2103 (JP); SATO, Keiko, Nishi-ku, Kobe-shi, Hyogo 651-2112 (JP)
(74) Representative: Webber, Philip Michael
(86) International application number: PCT/JP2001/001871
(87) International publication number: WO 2001/066736

(56) References cited:
- DATABASE EMBL [Online] 20 May 1999 (1999-05-20) "Homo sapiens mRNA, cDNA DKFZp566B0846" retrieved from EBI Database accession no. AL050071 XP002268762
- DATABASE EMBL [Online] 26 April 1999 (1999-04-26) "Stratagene mouse testis vi73c03 " retrieved from EBI Database accession no. AI614172 XP002268763
- DATABASE EMBL [Online] 16 March 1999 (1999-03-16) "Stratagene mouse testis m146h10 EST" retrieved from EBI Database accession no. AI428160 XP002268764
- DATABASE EMBL [Online] 1 November 1999 (1999-11-01) "Hypothetical protein fragment" retrieved from EBI Database accession no. Q9Y412 XP002268765
- AOKI JUNKEN ET AL: "Mouse homolog of poliovirus receptor-related gene 2 product, mPRR2, mediates homophilic cell aggregation" EXPERIMENTAL CELL RESEARCH, vol. 235, no. 2, 1997, pages 374-384, XP002268759 ISSN: 0014-4827
- MORRISON M E ET AL: "MOLECULAR CLONING AND EXPRESSION OF A MURINE HOMOLOG OF THE HUMAN POLIOVIRUS RECEPTOR GENE" JOURNAL OF VIROLOGY, vol. 66, no. 5, 1992, pages 2807-2813, XP002268760 ISSN: 0022-538X
- REYMOND N ET AL: "Human nectin3/PRR3: a novel member of the PVR/PRR/nectin family that interacts with afadin" GENE, ELSEVIER BIOMEDICAL PRESS. AMSTERDAM, NL, vol. 255, no. 2, 19 September 2000 (2000-09-19), pages 347-355, XP004217649 ISSN: 0378-1119
- SATOH-HORIKAWA K ET AL: "Nectin-3, a New Member of Immunoglobulin-like Cell Adhesion Molecules That Shows Homophilic and Heterophilic Cell-Cell Adhesion Activities" JOURNAL OF BIOLOGICAL CHEMISTRY, AMERICAN SOCIETY OF BIOLOGICAL CHEMISTS, BALTIMORE, MD, US, vol. 275, no. 14, 7 April 2000 (2000-04-07), pages 10291-10299, XP002198184 ISSN: 0021-9258
- DATABASE EMBL [Online] 7 August 2000 (2000-08-07) "Homo sapiens nectin 3 mRNA, complete cds." retrieved from EBI Database accession no. AF282874 XP002268766
- LOPEZ MARC ET AL: "The human poliovirus receptor related 2 protein is a new hematopoietic/endothelial homophilic adhesion molecule" BLOOD, vol. 92, no. 12, 15 December 1998 (1998-12-15), pages 4602-4611, XP002268761 ISSN: 0006-4971
- TAKAHASHI K. ET AL.: 'An immunoglobulin-like cell adhesion molecule recruited to cadherin-based adherens junctions through interaction with Afadin, a PDZ domain-containing protein' J. CELL. BIOL. vol. 145, no. 3, 1999, pages 539 - 549, XP002941478
- MIYAHARA M. ET AL.: 'Interaction of nection with afadin is necessary for its clustering at cell-cell contact sites but not for its cis dimerization or trans interaction' J. BIOL. CHEM. vol. 275, no. 1, 2000, pages 613 - 618, XP002941479
- ASAKURA T. ET AL.: 'Similar and differential behaviour between the nection-afadin-ponsin and cadherin-catenin systems during the formation and disruption of the polarized junctional alignment in epithelial cells' GENES CELLS vol. 4, no. 10, 1999, pages 573 - 581, XP002941480

### SEQUENCE LISTING

<110> Japan Science and Technology Corporation
<120> Protein Nectine-3
<130> 00-F-039PCT/YS
<140> PCT/JP01/01871
   <141> 2001-03-09
<150> JP 2000-65595
   <151> 2000-03-09
<160> 7
<170> PatentIn Ver. 2.1
<210>1
   <211> 1650
   <212> DNA
   <213> Mouse
<220>
   <221> CDS
   <222> (1).. (1650)
<300>
   <301> Satoh-Horikawa K et al.
   <302> Nectin-3, a new member of immunoglobulin-like cell adhesion molecules that shows homophilic and heterophilic cell-cell adhesion activities.
   <303> J. Biol. Chem.
   <304> 275
   <305> 14
   <306> 10291-10299
   <307> 2000-04-07
<400> 1
   atg gcg cgg acc ccg ggc ccg gcc ccg ttg tgt cct gga ggc ggc aaa    48
   Met Ala Arg Thr Pro Gly Pro Ala Pro Leu Cys Pro Gly Gly Gly Lys
    1               5                  10                  15
   gca caa ctt tcc tcg gcg ttt cct ccc gcg gcc gga ctg ctg ctg ccg    96
   Ala Gln Leu Ser Ser Ala Phe Pro Pro Ala Ala Gly Leu Leu Leu Pro
               20                  25                  30
   gcc ccg acg ccg ccg ccg ctg ctg ctg ctg ctt att ccc ctg ctt ctc   144
   Ala Pro Thr Pro Pro Pro Leu Leu Leu Leu Leu Ile Pro Leu Leu Leu
           35                  40                  45
   ttc tcc cgg ctc tgt ggt gcc tta gct gga tca att att gtg gag cca   192
   Phe Ser Arg Leu Cys Gly Ala Leu Ala Gly Ser Ile Ile Val Glu Pro
       50                  55                  60
   cat gtc aca gca gtg tgg gga aag aat gtt tca ttg aag tgt tta att   240
   His Val Thr Ala Val Trp Gly Lys Asn Val Ser Leu Lys Cys Leu Ile
   65                  70                  75                  80
   gaa gtg aat gaa act ata acc cag atc tca tgg gag aag ata cat ggc   288
   Glu Val Asn Glu Thr Ile Thr Gln Ile Ser Trp Glu Lys Ile His Gly
                   85                  90                  95
   aaa agt aca cag act gtt gca gtt cat cat cct cag tat gga ttc tct   336
   Lys Ser Thr Gln Thr Val Ala Val His His Pro Gln Tyr Gly Phe Ser
               100                 105                 110
   gtt caa gga gat tat cag gga aga gtc ttg ttt aaa aac tat tca ctt   384
   Val Gln Gly Asp Tyr Gln Gly Arg Val Leu Phe Lys Asn Tyr Ser Leu
   115 120 125
   aat gat gca aca att act ctg cat aac ata ggc ttc tca gat tct gga 432
   Asn Asp Ala Thr Ile Thr Leu His Asn Ile Gly Phe Ser Asp Ser Gly
   130 135 140
   aaa tat ata tgc aaa gcc gtt aca ttc cca ctt gga aat gct cag tcc 480
   Lys Tyr Ile Cys Lys Ala Val Thr Phe Pro Leu Gly Asn Ala Gln Ser
   145 150 155 160
   tct aca aca gtg act gtg tta gtt gaa ccc aca gtg agc ctg ata aaa 528
   Ser Thr Thr Val Thr Val Leu Val Glu Pro Thr Val Ser Leu Ile Lys
   165 170 175
   ggg ccg gat tct tta att gat gga ggg aat gag aca gta gca gcc gtt 576
   Gly Pro Asp Ser Leu Ile Asp Gly Gly Asn Glu Thr Val Ala Ala Val
   180 185 190
   tgt gta gca gcc act gga aag cca gtc gca cag att gac tgg gaa ggt 624
   Cys Val Ala Ala Thr Gly Lys Pro Val Ala Gln Ile Asp Trp Glu Gly
   195 200 205
   gat ctt ggt gaa atg gaa tct agt aca act tct ttt cct aat gaa aca 672
   Asp Leu Gly Glu Met Glu Ser Ser Thr Thr Ser Phe Pro Asn Glu Thr
   210 215 220
   gca acg att gtc agc caa tac aag ctg ttt ccc aca aga ttt gct cga 720
   Ala Thr Ile Val Ser Gln Tyr Lys Leu Phe Pro Thr Arg Phe Ala Arg
   225 230 235 240
   gga agg cga att act tgt gtt gta aaa cat cca gcc tta gaa aag gac 768
   Gly Arg Arg Ile Thr Cys Val Val Lys His Pro Ala Leu Glu Lys Asp
   245 250 255
   att cgc tac tct ttc ata cta gac ata cag tat gct cct gaa gtt tca 816
   Ile Arg Tyr Ser Phe Ile Leu Asp Ile Gln Tyr Ala Pro Glu Val Ser
   260 265 270
   gta aca gga tat gat gga aat tgg ttc gtg gga aga aaa ggt gtt aac 864
   Val Thr Gly Tyr Asp Gly Asn Trp Phe Val Gly Arg Lys Gly Val Asn
   275 280 285
   ctc aag tgt aat gct gat gca aac cct cca ccc ttc aag tcc gtg tgg 912
   Leu Lys Cys Asn Ala Asp Ala Asn Pro Pro Pro Phe Lys Ser Val Trp
   290 295 300
   agc agg ttg gat gga caa tgg cct gat ggt tta ttg gcg tca gat aat 960
   Ser Arg Leu Asp Gly Gln Trp Pro Asp Gly Leu Leu Ala Ser Asp Asn
   305 310 315 320
   act ctt cat ttt gtc cat cca ttg act gtc aat tat tct ggc gtt tat 1008
   Thr Leu His Phe Val His Pro Leu Thr Val Asn Tyr Ser Gly Val Tyr
   325 330 335
   gtc tgt aaa gta tca aat tcc ctt ggt caa aga agt gat caa aag gtt 1056
   Val Cys Lys Val Ser Asn Ser Leu Gly Gln Arg Ser Asp Gln Lys Val
   340 345 350
   atc tac att tca gat cct cct acc acc acc acc ctt cag ccg aca gtt 1104
   Ile Tyr Ile Ser Asp Pro Pro Thr Thr Thr Thr Leu Gln Pro Thr Val
   355 360 365
   cag tgg cat tcc tca cct gct gac gtc cag gat ata gca aca gag cat 1152
   Gln Trp His Ser Ser Pro Ala Asp Val Gln Asp Ile Ala Thr Glu His
   370 375 380
   aaa aaa ttg ccc ttt cct ttg tca act ttg gca aca ctt aag gat gac 1200
   Lys Lys Leu Pro Phe Pro Leu Ser Thr Leu Ala Thr Leu Lys Asp Asp
   385 390 395 400
   aca att ggc acc atc att gct agt gta gtg ggt ggg gct ctc ttc tta 1248
   Thr Ile Gly Thr Ile Ile Ala Ser Val Val Gly Gly Ala Leu Phe Leu
   405 410 415
   gtg ctt gtg agc att tta gct ggg gta ttc tgc tat agg aga cga cgg 1296
   Val Leu Val Ser Ile Leu Ala Gly Val Phe Cys Tyr Arg Arg Arg Arg
   420 425 430
   acg ttt cgt gga gac tac ttt gcc aaa aac tac att cca cca tca gac 1344
   Thr Phe Arg Gly Asp Tyr Phe Ala Lys Asn Tyr Ile Pro Pro Ser Asp
   435 440 445
   atg cag aaa gaa tca cag att gat gtt ctt cac cag gat gag ctg gat 1392
   Met Gln Lys Glu Ser Gln Ile Asp Val Leu His Gln Asp Glu Leu Asp
   450 455 460
   tct tac cca gac agt gta aaa aag gaa aac aaa aat cca gta aac aac 1440
   Ser Tyr Pro Asp Ser Val Lys Lys Glu Asn Lys Asn Pro Val Asn Asn
   465 470 475 480
   ctg atc cgc aaa gac tac tta gag gag cct gag aaa act cag tgg aat 1488
   Leu Ile Arg Lys Asp Tyr Leu Glu Glu Pro Glu Lys Thr Gln Trp Asn
   485 490 495
   aat gta gag aac ctc act agg ttt gaa aga ccg atg gat tac tat gaa 1536
   Asn Val Glu Asn Leu Thr Arg Phe Glu Arg Pro Met Asp Tyr Tyr Glu
   500 505 510
   gat cta aaa atg gga atg aag ttt gtc agt gat gaa cgc tac aat gaa 1584
   Asp Leu Lys Met Gly Met Lys Phe Val Ser Asp Glu Arg Tyr Asn Glu
   515 520 525
   agt gaa gat ggt ttg gtt tct cat gta gat ggc tcc gta att tcc agg 1632
   Ser Glu Asp Gly Leu Val Ser His Val Asp Gly Ser Val Ile Ser Arg
   530 535 540
   agg gag tgg tat gtc taa 1650
   Arg Glu Trp Tyr Val
   545 550
<210> 2
   <211> 549
   <212> PRT
   <213> Mouse
<400> 2
   Met Ala Arg Thr Pro Gly Pro Ala Pro Leu Cys Pro Gly Gly Gly Lys
   1 5 10 15
   Ala Gln Leu Ser Ser Ala Phe Pro Pro Ala Ala Gly Leu Leu Leu Pro
   20 25 30
   Ala Pro Thr Pro Pro Pro Leu Leu Leu Leu Leu Ile Pro Leu Leu Leu
   35 40 45
   Phe Ser Arg Leu Cys Gly Ala Leu Ala Gly Ser Ile Ile Val Glu Pro
   50 55 60
   His Val Thr Ala Val Trp Gly Lys Asn Val Ser Leu Lys Cys Leu Ile
   65 70 75 80
   Glu Val Asn Glu Thr Ile Thr Gln Ile Ser Trp Glu Lys Ile His Gly
   85 90 95
   Lys Ser Thr Gln Thr Val Ala Val His His Pro Gln Tyr Gly Phe Ser
   100 105 110
   Val Gln Gly Asp Tyr Gln Gly Arg Val Leu Phe Lys Asn Tyr Ser Leu
   115 120 125
   Asn Asp Ala Thr Ile Thr Leu His Asn Ile Gly Phe Ser Asp Ser Gly
   130 135 140
   Lys Tyr Ile Cys Lys Ala Val Thr Phe Pro Leu Gly Asn Ala Gln Ser
   145 150 155 160
   Ser Thr Thr Val Thr Val Leu Val Glu Pro Thr Val Ser Leu Ile Lys
   165 170 175
   Gly Pro Asp Ser Leu Ile Asp Gly Gly Asn Glu Thr Val Ala Ala Val
   180 185 190
   Cys Val Ala Ala Thr Gly Lys Pro Val Ala Gln Ile Asp Trp Glu Gly
   195 200 205
   Asp Leu Gly Glu Met Glu Ser Ser Thr Thr Ser Phe Pro Asn Glu Thr
   210 215 220
   Ala Thr Ile Val Ser Gln Tyr Lys Leu Phe Pro Thr Arg Phe Ala Arg
   225 230 235 240
   Gly Arg Arg Ile Thr Cys Val Val Lys His Pro Ala Leu Glu Lys Asp
   245 250 255
   Ile Arg Tyr Ser Phe Ile Leu Asp Ile Gln Tyr Ala Pro Glu Val Ser
   260 265 270
   Val Thr Gly Tyr Asp Gly Asn Trp Phe Val Gly Arg Lys Gly Val Asn
   275 280 285
   Leu Lys Cys Asn Ala Asp Ala Asn Pro Pro Pro Phe Lys Ser Val Trp
   290 295 300
   Ser Arg Leu Asp Gly Gln Trp Pro Asp Gly Leu Leu Ala Ser Asp Asn
   305 310 315 320
   Thr Leu His Phe Val His Pro Leu Thr Val Asn Tyr Ser Gly Val Tyr
   325 330 335
   Val Cys Lys Val Ser Asn Ser Leu Gly Gln Arg Ser Asp Gln Lys Val
   340 345 350
   Ile Tyr Ile Ser Asp Pro Pro Thr Thr Thr Thr Leu Gln Pro Thr Val
   355 360 365
   Gln Trp His Ser Ser Pro Ala Asp Val Gln Asp Ile Ala Thr Glu His
   370 375 380
   Lys Lys Leu Pro Phe Pro Leu Ser Thr Leu Ala Thr Leu Lys Asp Asp
   385 390 395 400
   Thr Ile Gly Thr Ile Ile Ala Ser Val Val Gly Gly Ala Leu Phe Leu
   405 410 415
   Val Leu Val Ser Ile Leu Ala Gly Val Phe Cys Tyr Arg Arg Arg Arg
   420 425 430
   Thr Phe Arg Gly Asp Tyr Phe Ala Lys Asn Tyr Ile Pro Pro Ser Asp
   435 440 445
   Met Gln Lys Glu Ser Gln Ile Asp Val Leu His Gln Asp Glu Leu Asp
   450 455 460
   Ser Tyr Pro Asp Ser Val Lys Lys Glu Asn Lys Asn Pro Val Asn Asn
   465 470 475 480
   Leu Ile Arg Lys Asp Tyr Leu Glu Glu Pro Glu Lys Thr Gln Trp Asn
   485 490 495
   Asn Val Glu Asn Leu Thr Arg Phe Glu Arg Pro Met Asp Tyr Tyr Glu
   500 505 510
   Asp Leu Lys Met Gly Met Lys Phe Val Ser Asp Glu Arg Tyr Asn Glu
   515 520 525
   Ser Glu Asp Gly Leu Val Ser His Val Asp Gly Ser Val Ile Ser Arg
   530 535 540
   Arg Glu Trp Tyr Val
   545
<210> 3
   <211> 1533
   <212> DNA
   <213> Mouse
<220>
   <221> CDS
   <222> (1).. (1533)
<300>
   <301> Satoh-Horikawa K et al.
   <302> Nectin-3, a new member of immunoglobulin-like cell adhesion molecules that shows homophilic and heterophilic cell-cell adhesion activities.
   <303> J. Biol. Chem.
   <304> 275
   <305> 14
   <306> 10291-10299
   <307> 2000-04-07
   <308> GenBank accession No. AF195834
   <309> 2000-04-13
<400> 3
   atg gcg cgg acc ccg ggc ccg gcc ccg ttg tgt cct gga ggc ggc aaa 48
   Met Ala Arg Thr Pro Gly Pro Ala Pro Leu Cys Pro Gly Gly Gly Lys
   1 5 10 15
   gca caa ctt tcc tcg gcg ttt cct ccc gcg gcc gga ctg ctg ctg ccg 96
   Ala Gln Leu Ser Ser Ala Phe Pro Pro Ala Ala Gly Leu Leu Leu Pro
   20 25 30
   gcc ccg acg ccg ccg ccg ctg ctg ctg ctg ctt att ccc ctg ctt ctc 144
   Ala Pro Thr Pro Pro Pro Leu Leu Leu Leu Leu Ile Pro Leu Leu Leu
   35 40 45
   ttc tcc cgg ctc tgt ggt gcc tta gct gga tca att att gtg gag cca 192
   Phe Ser Arg Leu Cys Gly Ala Leu Ala Gly Ser Ile Ile Val Glu Pro
   50 55 60
   cat gtc aca gca gtg tgg gga aag aat gtt tca ttg aag tgt tta att 240
   His Val Thr Ala Val Trp Gly Lys Asn Val Ser Leu Lys Cys Leu Ile
    65                  70                  75                  80
   gaa gtg aat gaa act ata acc cag atc tca tgg gag aag ata cat ggc     288
   Glu Val Asn Glu Thr Ile Thr Gln Ile Ser Trp Glu Lys Ile His Gly
                   85                  90                  95
   aaa agt aca cag act gtt gca gtt cat cat cct cag tat gga ttc tct     336
   Lys Ser Thr Gln Thr Val Ala Val His His Pro Gln Tyr Gly Phe Ser
               100                 105                 110
   gtt caa gga gat tat cag gga aga gtc ttg ttt aaa aac tat tca ctt     384
   Val Gln Gly Asp Tyr Gln Gly Arg Val Leu Phe Lys Asn Tyr Ser Leu
           115                 120                 125
   aat gat gca aca att act ctg cat aac ata ggc ttc tca gat tct gga     432
   Asn Asp Ala Thr Ile Thr Leu His Asn Ile Gly Phe Ser Asp Ser Gly
       130                 135                 140
   aaa tat ata tgc aaa gcc gtt aca ttc cca ctt gga aat gct cag tcc     480
   Lys Tyr Ile Cys Lys Ala Val Thr Phe Pro Leu Gly Asn Ala Gln Ser
   145                 150                 155                 160
   tct aca aca gtg act gtg tta gtt gaa ccc aca gtg agc ctg ata aaa     528
   Ser Thr Thr Val Thr Val Leu Val Glu Pro Thr Val Ser Leu Ile Lys
                   165                 170                 175
   ggg ccg gat tct tta att gat gga ggg aat gag aca gta gca gcc gtt     576
   Gly Pro Asp Ser Leu Ile Asp Gly Gly Asn Glu Thr Val Ala Ala Val
               180                 185                 190
   tgt gta gca gcc act gga aag cca gtc gca cag att gac tgg gaa ggt     624
   Cys Val Ala Ala Thr Gly Lys Pro Val Ala Gln Ile Asp Trp Glu Gly
           195                 200                 205
   gat ctt ggt gaa atg gaa tct agt aca act tct ttt cct aat gaa aca   672
   Asp Leu Gly Glu Met Glu Ser Ser Thr Thr Ser Phe Pro Asn Glu Thr
       210                 215                 220
   gca acg att gtc agc caa tac aag ctg ttt ccc aca aga ttt gct cga   720
   Ala Thr Ile Val Ser Gln Tyr Lys Leu Phe Pro Thr Arg Phe Ala Arg
   225                 230                 235                 240
   gga agg cga att act tgt gtt gta aaa cat cca gcc tta gaa aag gac   768
   Gly Arg Arg Ile Thr Cys Val Val Lys His Pro Ala Leu Glu Lys Asp
                   245                 250                 255
   att cgc tac tct ttc ata cta gac ata cag tat gct cct gaa gtt tca   816
   Ile Arg Tyr Ser Phe Ile Leu Asp Ile Gln Tyr Ala Pro Glu Val Ser
               260                 265                 270
   gta aca gga tat gat gga aat tgg ttc gtg gga aga aaa ggt gtt aac   864
   Val Thr Gly Tyr Asp Gly Asn Trp Phe Val Gly Arg Lys Gly Val Asn
           275                 280                 285
   ctc aag tgt aat gct gat gca aac cct cca ccc ttc aag tcc gtg tgg   912
   Leu Lys Cys Asn Ala Asp Ala Asn Pro Pro Pro Phe Lys Ser Val Trp
       290                 295                 300
   agc agg ttg gat gga caa tgg cct gat ggt tta ttg gcg tca gat aat   960
   Ser Arg Leu Asp Gly Gln Trp Pro Asp Gly Leu Leu Ala Ser Asp Asn
   305                 310                 315                 320
   act ctt cat ttt gtc cat cca ttg act gtc aat tat tct ggc gtt tat   1008
   Thr Leu His Phe Val His Pro Leu Thr Val Asn Tyr Ser Gly Val Tyr
                   325                 330                 335
   gtc tgt aaa gta tca aat tcc ctt ggt caa aga agt gat caa aag gtt 1056
   Val Cys Lys Val Ser Asn Ser Leu Gly Gln Arg Ser Asp Gln Lys Val
   340 345 350
   atc tac att tca gac atc ccg ctt acg cag acc tca tcc ata gca gtg 1104
   Ile Tyr Ile Ser Asp Ile Pro Leu Thr Gln Thr Ser Ser Ile Ala Val
   355 360 365
   gct gga gcc gtg att gga gct gtc ctg gcc ctc ttc atc atc acc gtc 1152
   Ala Gly Ala Val Ile Gly Ala Val Leu Ala Leu Phe Ile Ile Thr Val
   370 375 380
   ttt gtg act gtg ttg ctg acg cct cgg aaa aag aga ccg tcc tat ctt 1200
   Phe Val Thr Val Leu Leu Thr Pro Arg Lys Lys Arg Pro Ser Tyr Leu
   385 390 395 400
   gac aaa gta atc gac ctt cca cct aca cat aag cca ccc cct gta tat 1248
   Asp Lys Val Ile Asp Leu Pro Pro Thr His Lys Pro Pro Pro Val Tyr
   405 410 415
   gaa gaa cga att cct tct ctc cct cag aaa gac ctt ctt ggc cag act 1296
   Glu Glu Arg Ile Pro Ser Leu Pro Gln Lys Asp Leu Leu Gly Gln Thr
   420 425 430
   gaa cac ttg cct ttg cag act cag ttc aag gag aaa gga gct ggt ggt 1344
   Glu His Leu Pro Leu Gln Thr Gln Phe Lys Glu Lys Gly Ala Gly Gly
   435 440 445
   ctt cag ccc tct aat gga cca att agc agg aga ttt gac tat gag gat 1392
   Leu Gln Pro Ser Asn Gly Pro Ile Ser Arg Arg Phe Asp Tyr Glu Asp
   450 455 460
   gag agc aca atg caa gaa gat gga act cag cgc atg tgc ccc ctt tat 1440
   Glu Ser Thr Met Gln Glu Asp Gly Thr Gln Arg Met Cys Pro Leu Tyr
   465 470 475 480
   agc cag atg tgc cac caa gac cga agc cct cgc caa cat cac cca cgc 1488
   Ser Gln Met Cys His Gln Asp Arg Ser Pro Arg Gln His His Pro Arg
   485 490 495
   aac ccc gag aga ctc tac atc aac cca cga gaa cat tat gtg tga 1533
   Asn Pro Glu Arg Leu Tyr Ile Asn Pro Arg Glu His Tyr Val
   500 505 510
<210> 4
   <211> 510
   <212> PRT
   <213> Mouse
<400> 4
   Met Ala Arg Thr Pro Gly Pro Ala Pro Leu Cys Pro Gly Gly Gly Lys
   1 5 10 15
   Ala Gln Leu Ser Ser Ala Phe Pro Pro Ala Ala Gly Leu Leu Leu Pro
   20 25 30
   Ala Pro Thr Pro Pro Pro Leu Leu Leu Leu Leu Ile Pro Leu Leu Leu
   35 40 45
   Phe Ser Arg Leu Cys Gly Ala Leu Ala Gly Ser Ile Ile Val Glu Pro
   50 55 60
   His Val Thr Ala Val Trp Gly Lys Asn Val Ser Leu Lys Cys Leu Ile
   65 70 75 80
   Glu Val Asn Glu Thr Ile Thr Gln Ile Ser Trp Glu Lys Ile His Gly
   85 90 95
   Lys Ser Thr Gln Thr Val Ala Val His His Pro Gln Tyr Gly Phe Ser
   100 105 110
   Val Gln Gly Asp Tyr Gln Gly Arg Val Leu Phe Lys Asn Tyr Ser Leu
   115 120 125
   Asn Asp Ala Thr Ile Thr Leu His Asn Ile Gly Phe Ser Asp Ser Gly
   130 135 140
   Lys Tyr Ile Cys Lys Ala Val Thr Phe Pro Leu Gly Asn Ala Gln Ser
   145 150 155 160
   Ser Thr Thr Val Thr Val Leu Val Glu Pro Thr Val Ser Leu Ile Lys
   165 170 175
   Gly Pro Asp Ser Leu Ile Asp Gly Gly Asn Glu Thr Val Ala Ala Val
   180 185 190
   Cys Val Ala Ala Thr Gly Lys Pro Val Ala Gln Ile Asp Trp Glu Gly
   195 200 205
   Asp Leu Gly Glu Met Glu Ser Ser Thr Thr Ser Phe Pro Asn Glu Thr
   210 215 220
   Ala Thr Ile Val Ser Gln Tyr Lys Leu Phe Pro Thr Arg Phe Ala Arg
   225 230 235 240
   Gly Arg Arg Ile Thr Cys Val Val Lys His Pro Ala Leu Glu Lys Asp
   245 250 255
   Ile Arg Tyr Ser Phe Ile Leu Asp Ile Gln Tyr Ala Pro Glu Val Ser
   260 265 270
   Val Thr Gly Tyr Asp Gly Asn Trp Phe Val Gly Arg Lys Gly Val Asn
   275 280 285
   Leu Lys Cys Asn Ala Asp Ala Asn Pro Pro Pro Phe Lys Ser Val Trp
   290 295 300
   Ser Arg Leu Asp Gly Gln Trp Pro Asp Gly Leu Leu Ala Ser Asp Asn
   305 310 315 320
   Thr Leu His Phe Val His Pro Leu Thr Val Asn Tyr Ser Gly Val Tyr
   325 330 335
   Val Cys Lys Val Ser Asn Ser Leu Gly Gln Arg Ser Asp Gln Lys Val
   340 345 350
   Ile Tyr Ile Ser Asp Ile Pro Leu Thr Gln Thr Ser Ser Ile Ala Val
   355 360 365
   Ala Gly Ala Val Ile Gly Ala Val Leu Ala Leu Phe Ile Ile Thr Val
   370 375 380
   Phe Val Thr Val Leu Leu Thr Pro Arg Lys Lys Arg Pro Ser Tyr Leu
   385 390 395 400
   Asp Lys Val Ile Asp Leu Pro Pro Thr His Lys Pro Pro Pro Val Tyr
   405 410 415
   Glu Glu Arg Ile Pro Ser Leu Pro Gln Lys Asp Leu Leu Gly Gln Thr
   420 425 430
   Glu His Leu Pro Leu Gln Thr Gln Phe Lys Glu Lys Gly Ala Gly Gly
   435 440 445
   Leu Gln Pro Ser Asn Gly Pro Ile Ser Arg Arg Phe Asp Tyr Glu Asp
   450 455 460
   Glu Ser Thr Met Gln Glu Asp Gly Thr Gln Arg Met Cys Pro Leu Tyr
   465 470 475 480
   Ser Gln Met Cys His Gln Asp Arg Ser Pro Arg Gln His His Pro Arg
   485 490 495
   Asn Pro Glu Arg Leu Tyr Ile Asn Pro Arg Glu His Tyr Val
   500 505 510
<210> 5
   <211> 1317
   <212> DNA
   <213> Mouse
<220>
   <221> CDS
   <222> (1) .. (1317)
<300>
   <301> Satoh-Horikawa K. et at.
   <302> Nectin-3, a new member of immunoglobulin-like cell adhesion molecules that shows homophilic and heterophilic cell-cell adhesion activities.
   <303> J. Biol. Chem.
   <304> 275
   <305> 14
   <306> 10291-10299
   <307> 2000-04-07
   <308> GenBank accession No. AF195835
   <309> 2000-04-13
<400> 5
   atg gcg cgg acc ccg ggc ccg gcc ccg ttg tgt cct gga ggc ggc aaa    48
   Met Ala Arg Thr Pro Gly Pro Ala Pro Leu Cys Pro Gly Gly Gly Lys
    1               5                  10                  15
   gca caa ctt tcc tcg gcg ttt cct ccc gcg gcc gga ctg ctg ctg ccg    96
   Ala Gln Leu Ser Ser Ala Phe Pro Pro Ala Ala Gly Leu Leu Leu Pro
               20                  25                  30
   gcc ccg acg ccg ccg ccg ctg ctg ctg ctg ctt att ccc ctg ctt ctc   144
   Ala Pro Thr Pro Pro Pro Leu Leu Leu Leu Leu Ile Pro Leu Leu Leu
           35                  40                  45
   ttc tcc cgg ctc tgt ggt gcc tta gct gga tca att att gtg gag cca   192
   Phe Ser Arg Leu Cys Gly Ala Leu Ala Gly Ser Ile Ile Val Glu Pro
       50                  55                  60
   cat gtc aca gca gtg tgg gga aag aat gtt tca ttg aag tgt tta att   240
   His Val Thr Ala Val Trp Gly Lys Asn Val Ser Leu Lys Cys Leu Ile
   65                  70                  75                  80
   gaa gtg aat gaa act ata acc cag atc tca tgg gag aag ata cat ggc   288
   Glu Val Asn Glu Thr Ile Thr Gln Ile Ser Trp Glu Lys Ile His Gly
                   85                  90                  95
   aaa agt aca cag act gtt gca gtt cat cat cct cag tat gga ttc tct   336
   Lys Ser Thr Gln Thr Val Ala Val His His Pro Gln Tyr Gly Phe Ser
   100 105 110
   gtt caa gga gat tat cag gga aga gtc ttg ttt aaa aac tat tca ctt 384
   Val Gln Gly Asp Tyr Gln Gly Arg Val Leu Phe Lys Asn Tyr Ser Leu
   115 120 125
   aat gat gca aca att act ctg cat aac ata ggc ttc tca gat tct gga 432
   Asn Asp Ala Thr Ile Thr Leu His Asn Ile Gly Phe Ser Asp Ser Gly
   130 135 140
   aaa tat ata tgc aaa gcc gtt aca ttc cca ctt gga aat gct cag tcc 480
   Lys Tyr Ile Cys Lys Ala Val Thr Phe Pro Leu Gly Asn Ala Gln Ser
   145 150 155 160
   tct aca aca gtg act gtg tta gtt gaa ccc aca gtg agc ctg ata aaa 528
   Ser Thr Thr Val Thr Val Leu Val Glu Pro Thr Val Ser Leu Ile Lys
   165 170 175
   ggg ccg gat tct tta att gat gga ggg aat gag aca gta gca gcc gtt 576
   Gly Pro Asp Ser Leu Ile Asp Gly Gly Asn Glu Thr Val Ala Ala Val
   180 185 190
   tgt gta gca gcc act gga aag cca gtc gca cag att gac tgg gaa ggt 624
   Cys Val Ala Ala Thr Gly Lys Pro Val Ala Gln Ile Asp Trp Glu Gly
   195 200 205
   gat ctt ggt gaa atg gaa tct agt aca act tct ttt cct aat gaa aca 672
   Asp Leu Gly Glu Met Glu Ser Ser Thr Thr Ser Phe Pro Asn Glu Thr
   210 215 220
   gca acg att gtc agc caa tac aag ctg ttt ccc aca aga ttt gct cga 720
   Ala Thr Ile Val Ser Gln Tyr Lys Leu Phe Pro Thr Arg Phe Ala Arg
   225 230 235 240
   gga agg cga att act tgt gtt gta aaa cat cca gcc tta gaa aag gac 768
   Gly Arg Arg Ile Thr Cys Val Val Lys His Pro Ala Leu Glu Lys Asp
   245 250 255
   att cgc tac tct ttc ata cta gac ata cag tat gct cct gaa gtt tca 816
   Ile Arg Tyr Ser Phe Ile Leu Asp Ile Gln Tyr Ala Pro Glu Val Ser
   260 265 270
   gta aca gga tat gat gga aat tgg ttc gtg gga aga aaa ggt gtt aac 864
   Val Thr Gly Tyr Asp Gly Asn Trp Phe Val Gly Arg Lys Gly Val Asn
   275 280 285
   ctc aag tgt aat gct gat gca aac cct cca ccc ttc aag tcc gtg tgg 912
   Leu Lys Cys Asn Ala Asp Ala Asn Pro Pro Pro Phe Lys Ser Val Trp
   290 295 300
   agc agg ttg gat gga caa tgg cct gat ggt tta ttg gcg tca gat aat 960
   Ser Arg Leu Asp Gly Gln Trp Pro Asp Gly Leu Leu Ala Ser Asp Asn
   305 310 315 320
   act ctt cat ttt gtc cat cca ttg act gtc aat tat tct ggc gtt tat 1008
   Thr Leu His Phe Val His Pro Leu Thr Val Asn Tyr Ser Gly Val Tyr
   325 330 335
   gtc tgt aaa gta tca aat tcc ctt ggt caa aga agt gat caa aag gtt 1056
   Val Cys Lys Val Ser Asn Ser Leu Gly Gln Arg Ser Asp Gln Lys Val
   340 345 350
   atc tac att tca gac atc ccg ctt acg cag acc tca tcc ata gca gtg 1104
   Ile Tyr Ile Ser Asp Ile Pro Leu Thr Gln Thr Ser Ser Ile Ala Val
   355 360 365
   gct gga gcc gtg att gga gct gtc ctg gcc ctc ttc atc atc acc gtc 1152
   Ala Gly Ala Val Ile Gly Ala Val Leu Ala Leu Phe Ile Ile Thr Val
   370 375 380
   ttt gtg act gtg ttg ctg acg cct cgg aaa aag aga ccg tcc tat ctt 1200
   Phe Val Thr Val Leu Leu Thr Pro Arg Lys Lys Arg Pro Ser Tyr Leu
   385 390 395 400
   gac aaa gta atc gac ctt cca cct aca cat aag cca ccc cct gta tat 1248
   Asp Lys Val Ile Asp Leu Pro Pro Thr His Lys Pro Pro Pro Val Tyr
   405 410 415
   gaa gaa cga att cct tct ctc cct cag aaa gac ctt ctt ggc cag gta 1296
   Glu Glu Arg Ile Pro Ser Leu Pro Gln Lys Asp Leu Leu Gly Gln Val
   420 425 430
   cgt gct ctc gaa gac act taa 1317
   Arg Ala Leu Glu Asp Thr
   435
<210> 6
   <211> 438
   <212> PRT
   <213> Mouse
<400> 6
   Met Ala Arg Thr Pro Gly Pro Ala Pro Leu Cys Pro Gly Gly Gly Lys
   1 5 10 15
   Ala Gln Leu Ser Ser Ala Phe Pro Pro Ala Ala Gly Leu Leu Leu Pro
   20 25 30
   Ala Pro Thr Pro Pro Pro Leu Leu Leu Leu Leu Ile Pro Leu Leu Leu
   35 40 45
   Phe Ser Arg Leu Cys Gly Ala Leu Ala Gly Ser Ile Ile Val Glu Pro
   50 55 60
   His Val Thr Ala Val Trp Gly Lys Asn Val Ser Leu Lys Cys Leu Ile
   65 70 75 80
   Glu Val Asn Glu Thr Ile Thr Gln Ile Ser Trp Glu Lys Ile His Gly
   85 90 95
   Lys Ser Thr Gln Thr Val Ala Val His His Pro Gln Tyr Gly Phe Ser
   100 105 110
   Val Gln Gly Asp Tyr Gln Gly Arg Val Leu Phe Lys Asn Tyr Ser Leu
   115 120 125
   Asn Asp Ala Thr Ile Thr Leu His Asn Ile Gly Phe Ser Asp Ser Gly
   130 135 140
   Lys Tyr Ile Cys Lys Ala Val Thr Phe Pro Leu Gly Asn Ala Gln Ser
   145 150 155 160
   Ser Thr Thr Val Thr Val Leu Val Glu Pro Thr Val Ser Leu Ile Lys
   165 170 175
   Gly Pro Asp Ser Leu Ile Asp Gly Gly Asn Glu Thr Val Ala Ala Val
   180 185 190
   Cys Val Ala Ala Thr Gly Lys Pro Val Ala Gln Ile Asp Trp Glu Gly
   195 200 205
   Asp Leu Gly Glu Met Glu Ser Ser Thr Thr Ser Phe Pro Asn Glu Thr
   210 215 220
   Ala Thr Ile Val Ser Gln Tyr Lys Leu Phe Pro Thr Arg Phe Ala Arg
   225 230 235 240
   Gly Arg Arg Ile Thr Cys Val Val Lys His Pro Ala Leu Glu Lys Asp
   245 250 255
   Ile Arg Tyr Ser Phe Ile Leu Asp Ile Gln Tyr Ala Pro Glu Val Ser
   260 265 270
   Val Thr Gly Tyr Asp Gly Asn Trp Phe Val Gly Arg Lys Gly Val Asn
   275 280 285
   Leu Lys Cys Asn Ala Asp Ala Asn Pro Pro Pro Phe Lys Ser Val Trp
   290 295 300
   Ser Arg Leu Asp Gly Gln Trp Pro Asp Gly Leu Leu Ala Ser Asp Asn
   305 310 315 320
   Thr Leu His Phe Val His Pro Leu Thr Val Asn Tyr Ser Gly Val Tyr
   325 330 335
   Val Cys Lys Val Ser Asn Ser Leu Gly Gln Arg Ser Asp Gln Lys Val
   340 345 350
   Ile Tyr Ile Ser Asp Ile Pro Leu Thr Gln Thr Ser Ser Ile Ala Val
   355 360 365
   Ala Gly Ala Val Ile Gly Ala Val Leu Ala Leu Phe Ile Ile Thr Val
   370 375 380
   Phe Val Thr Val Leu Leu Thr Pro Arg Lys Lys Arg Pro Ser Tyr Leu
   385 390 395 400
   Asp Lys Val Ile Asp Leu Pro Pro Thr His Lys Pro Pro Pro Val Tyr
   405 410 415
   Glu Glu Arg Ile Pro Ser Leu Pro Gln Lys Asp Leu Leu Gly Gln Val
   420 425 430
   Arg Ala Leu Glu Asp Thr
   435
<210> 7
   <211> 2178
   <212> DNA
   <213> Mouse
<220>
   <221> CDS
   <222> (197) .. (1846)
<300>
   <301> Satoh-Horikawa K. et al.
   <302> Nectin-3, a new member of immunoglobulin-like cell adhesion molecules that shows homophilic and heterophilic cell-cell adhesion activities.
   <303> J. Biol. Chem.
   <304> 275
   <305> 14
   <306> 10291-10299
   <307> 2000-04-07
   <308> GenBank accession No. AF195833
   <309> 2000-04-13
<400> 7
   gaattcggca cgagcgacgg cggagtcgag gcagccgcga gcgctcggcc gagtggcggc 60
   gggcggcgac ggcgcaggag ccgggggttg aggacacgcg cgctggccct tccgcgccgc 120
   ggccgccgcc gccgccgcca cccagagcct gaggcgccgg ggcgcgggcg agcgggtggg 180
   ccggggcaag gcgggc atg gcg cgg acc ccg ggc ccg gcc ccg ttg tgt cct 232
                     Met Ala Arg Thr Pro Gly Pro Ala Pro Leu Cys Pro
                      1               5                  10
   gga ggc ggc aaa gca caa ctt tcc tcg gcg ttt cct ccc gcg gcc gga   280
   Gly Gly Gly Lys Ala Gln Leu Ser Ser Ala Phe Pro Pro Ala Ala Gly
            15                  20                  25
   ctg ctg ctg ccg gcc ccg acg ccg ccg ccg ctg ctg ctg ctg ctt att   328
   Leu Leu Leu Pro Ala Pro Thr Pro Pro Pro Leu Leu Leu Leu Leu Ile
        30                  35                  40
   ccc ctg ctt ctc ttc tcc cgg ctc tgt ggt gcc tta gct gga tca att   376
   Pro Leu Leu Leu Phe Ser Arg Leu Cys Gly Ala Leu Ala Gly Ser Ile
    45                  50                  55                  60
   att gtg gag cca cat gtc aca gca gtg tgg gga aag aat gtt tca ttg   424
   Ile Val Glu Pro His Val Thr Ala Val Trp Gly Lys Asn Val Ser Leu
   65 70 75
   aag tgt tta att gaa gtg aat gaa act ata acc cag atc tca tgg gag 472
   Lys Cys Leu Ile Glu Val Asn Glu Thr Ile Thr Gln Ile Ser Trp Glu
   80 85 90
   aag ata cat ggc aaa agt aca cag act gtt gca gtt cat cat cct cag 520
   Lys Ile His Gly Lys Ser Thr Gln Thr Val Ala Val His His Pro Gln
   95 100 105
   tat gga ttc tct gtt caa gga gat tat cag gga aga gtc ttg ttt aaa 568
   Tyr Gly Phe Ser Val Gln Gly Asp Tyr Gln Gly Arg Val Leu Phe Lys
   110 115 120
   aac tat tca ctt aat gat gca aca att act ctg cat aac ata ggc ttc 616
   Asn Tyr Ser Leu Asn Asp Ala Thr Ile Thr Leu His Asn Ile Gly Phe
   125 130 135 140
   tca gat tct gga aaa tat ata tgc aaa gcc gtt aca ttc cca ctt gga 664
   Ser Asp Ser Gly Lys Tyr Ile Cys Lys Ala Val Thr Phe Pro Leu Gly
   145 150 155
   aat gct cag tcc tct aca aca gtg act gtg tta gtt gaa ccc aca gtg 712
   Asn Ala Gln Ser Ser Thr Thr Val Thr Val Leu Val Glu Pro Thr Val
   160 165 170
   agc ctg ata aaa ggg ccg gat tct tta att gat gga ggg aat gag aca 760
   Ser Leu Ile Lys Gly Pro Asp Ser Leu Ile Asp Gly Gly Asn Glu Thr
   175 180 185
   gta gca gcc gtt tgt gta gca gcc act gga aag cca gtc gca cag att 808
   Val Ala Ala Val Cys Val Ala Ala Thr Gly Lys Pro Val Ala Gln Ile
   190 195 200
   gac tgg gaa ggt gat ctt ggt gaa atg gaa tct agt aca act tct ttt 856
   Asp Trp Glu Gly Asp Leu Gly Glu Met Glu Ser Ser Thr Thr Ser Phe
   205 210 215 220
   cct aat gaa aca gca acg att gtc agc caa tac aag ctg ttt ccc aca 904
   Pro Asn Glu Thr Ala Thr Ile Val Ser Gln Tyr Lys Leu Phe Pro Thr
   225 230 235
   aga ttt gct cga gga agg cga att act tgt gtt gta aaa cat cca gcc 952
   Arg Phe Ala Arg Gly Arg Arg Ile Thr Cys Val Val Lys His Pro Ala
   240 245 250
   tta gaa aag gac att cgc tac tct ttc ata cta gac ata cag tat gct 1000
   Leu Glu Lys Asp Ile Arg Tyr Ser Phe Ile Leu Asp Ile Gln Tyr Ala
   255 260 265
   cct gaa gtt tca gta aca gga tat gat gga aat tgg ttc gtg gga aga 1048
   Pro Glu Val Ser Val Thr Gly Tyr Asp Gly Asn Trp Phe Val Gly Arg
   270 275 280
   aaa ggt gtt aac ctc aag tgt aat gct gat gca aac cct cca ccc ttc 1096
   Lys Gly Val Asn Leu Lys Cys Asn Ala Asp Ala Asn Pro Pro Pro Phe
   285 290 295 300
   aag tcc gtg tgg agc agg ttg gat gga caa tgg cct gat ggt tta ttg 1144
   Lys Ser Val Trp Ser Arg Leu Asp Gly Gln Trp Pro Asp Gly Leu Leu
   305 310 315
   gcg tca gat aat act ctt cat ttt gtc cat cca ttg act gtc aat tat 1192
   Ala Ser Asp Asn Thr Leu His Phe Val His Pro Leu Thr Val Asn Tyr
   320 325 330
   tct ggc gtt tat gtc tgt aaa gta tca aat tcc ctt ggt caa aga agt 1240
   Ser Gly Val Tyr Val Cys Lys Val Ser Asn Ser Leu Gly Gln Arg Ser
   335 340 345
   gat caa aag gtt atc tac att tca gat cct cct acc acc acc acc ctt 1288
   Asp Gln Lys Val Ile Tyr Ile Ser Asp Pro Pro Thr Thr Thr Thr Leu
   350 355 360
   cag ccg aca gtt cag tgg cat tcc tca cct gct gac gtc cag gat ata 1336
   Gln Pro Thr Val Gln Trp His Ser Ser Pro Ala Asp Val Gln Asp Ile
   365 370 375 380
   gca aca gag cat aaa aaa ttg ccc ttt cct ttg tca act ttg gca aca 1384
   Ala Thr Glu His Lys Lys Leu Pro Phe Pro Leu Ser Thr Leu Ala Thr
   385 390 395
   ctt aag gat gac aca att ggc acc atc att gct agt gta gtg ggt ggg 1432
   Leu Lys Asp Asp Thr Ile Gly Thr Ile Ile Ala Ser Val Val Gly Gly
   400 405 410
   gct ctc ttc tta gtg ctt gtg agc att tta gct ggg gta ttc tgc tat 1480
   Ala Leu Phe Leu Val Leu Val Ser Ile Leu Ala Gly Val Phe Cys Tyr
   415 420 425
   agg aga cga cgg acg ttt cgt gga gac tac ttt gcc aaa aac tac att 1528
   Arg Arg Arg Arg Thr Phe Arg Gly Asp Tyr Phe Ala Lys Asn Tyr Ile
   430 435 440
   cca cca tca gac atg cag aaa gaa tca cag att gat gtt ctt cac cag 1576
   Pro Pro Ser Asp Met Gln Lys Glu Ser Gln Ile Asp Val Leu His Gln
   445 450 455 460
   gat gag ctg gat tct tac cca gac agt gta aaa aag gaa aac aaa aat 1624
   Asp Glu Leu Asp Ser Tyr Pro Asp Ser Val Lys Lys Glu Asn Lys Asn
   465 470 475
   cca gta aac aac ctg atc cgc aaa gac tac tta gag gag cct gag aaa 1672
   Pro Val Asn Asn Leu Ile Arg Lys Asp Tyr Leu Glu Glu Pro Glu Lys
   480 485 490
   act cag tgg aat aat gta gag aac ctc act agg ttt gaa aga ccg atg 1720
   Thr Gln Trp Asn Asn Val Glu Asn Leu Thr Arg Phe Glu Arg Pro Met
   495 500 505
   gat tac tat gaa gat cta aaa atg gga atg aag ttt gtc agt gat gaa 1768
   Asp Tyr Tyr Glu Asp Leu Lys Met Gly Met Lys Phe Val Ser Asp Glu
   510 515 520
   cgc tac aat gaa agt gaa gat ggt ttg gtt tct cat gta gat ggc tcc 1816
   Arg Tyr Asn Glu Ser Glu Asp Gly Leu Val Ser His Val Asp Gly Ser
   525 530 535 540
   gta att tcc agg agg gag tgg tat gtc taa cagccactga cgcgacttca 1866
   Val Ile Ser Arg Arg Glu Trp Tyr Val
   545 550
   ctatgtacaa ggtttcattc acactagttg accattttca gattgttcat actttttctt 1926
   gaggaagaat aagctttttc aagttgattt cgagcttact ttttatattc tgatctgaca 1986
   aatgaaaatg taaaacctgg gttcaatgta tctgagctgc tttacagttt tcactgctat 2046
   actactgtct caagatttaa attctaatgc agagtacttt attggtctga ggcacacagg 2106
   taagaaagat gtcaacgtta aatgtatgac gtttttggta caaaaattaa aaaaaaaaaa 2166
   aaaaaactcg ag 2178

## Description

### TECHNICAL FIELD

The invention of this application relates to a novel protein nectin-3 that participates in cadherin-based cell-cell adherens junctions, and to gene-engineering materials for obtaining and utilizing the protein.

### BACKGROUND ART

Cell-cell adhesion systems to be formed by transmembrane proteins, such as adhesion molecules, receptors and channels play an important role in various cell-level phenomena such as cell-cell adhesion, cell movement and cell morphology determination in animal individuals. Above all, cell-cell adherens junctions (AJs) bear a role indispensable for histocompatibility. Evidence is increasing to say that AJs further participate in controlling cell propagation and morphologic tissue formation, in addition to their mechanistic role as above. It has become clarified that many F-actin-binding proteins play a role as a linker to link actin cytoskeletons to adhesion molecules. However, the molecule-level understanding of AJs is insufficient, and it is not clear as to which molecules may bind actin cytoskeletons to cell membranes.

To clarify this, the inventors of this application have isolated some novel F-actin-binding proteins from rats' brains, analyzed the structure of the protein especially specific to neurocytes and abundantly existing in synapses, and named it "neurabin", for which the applicant already filed a patent application (Japanese Patent Application No. 276784/1998). Further, the inventors of this application have individually isolated 1-afadin, a novel F-actin-binding protein, and ponsin, a protein that binds with 1-afadin, for both of which the applicant filed patent applications (for 1-afadin, Japanese Patent Application No. 89572/ 1999; for ponsin, Japanese Patent Application No. 174687/1999). Still further, the inventors of this application have identified novel proteins, nectin-1α, 1β and nectin-2α, 2δ that function for cadherin-based AJ formation along with afadin (J. Cell Biol., 145:539-549, 1999). They have found that these nectin-1, -2 are Ca²⁺-independent immunoglobulin-like adhesion molecules and act on AJ formation in one system along with afadin and ponsin (Genes Cells 4:573-581, 1999; J. Biol. Chem., 275:613-618,2000).

Clarifying the molecular mechanism of cell-cell adhesion will make it possible to clarify, for example, the mechanism of humectation and metastasis of carcinoma, and is expected to be applicable to diagnosis of carcinoma for its malignancy and to a method for treating cases with carcinoma and also to development of medicines for carcinoma. For clarifying it, it is necessary to clarify all the details of the molecules that participate in cell-cell adhesion.

The invention of this application has been made in consideration of the current situation as above, and its one object is to provide a novel protein that participates in cell-cell adherens junctions.

Another object of the invention is to provide a gene-engineering material for producing the protein.

### DISCLOSURE OF THE INVENTION

To solve the problems noted above, this application provides a protein nectin-3 having the amino acid sequence of any of SEQ ID NO: 2, 4 or 6.

The protein nectin-3 is derived from mice, including three splicing variants expressed by mouse genomic gene (these proteins are hereinafter referred to as nectin-3α, nectin-3β, nectin 3γ).

This application also provides a polynucleotide that encodes the protein nectin-3. The polynucleotide includes genomic DNA, mRNAs and cDNAs.

This application further provides cDNAs each encoding the three types of nectin-3, or that is, polynucleotides each having the base sequence of any of SEQ ID NO:1,3 or 5.

This application still further provides a recombinant vector having any of these polynucleotides.

This application still further provides an antibody against any of the three types of protein nectin-3.

Like nectin-1 and nectin-2, the protein nectin-3 of this invention is common to all mammals, existing in any and every mammal. Therefore, the protein nectin-3 and the polynucleotide encoding it are not limited to only mouse-derived ones.

### BRIEF DESCRIPTION OF THE DRAWINGS

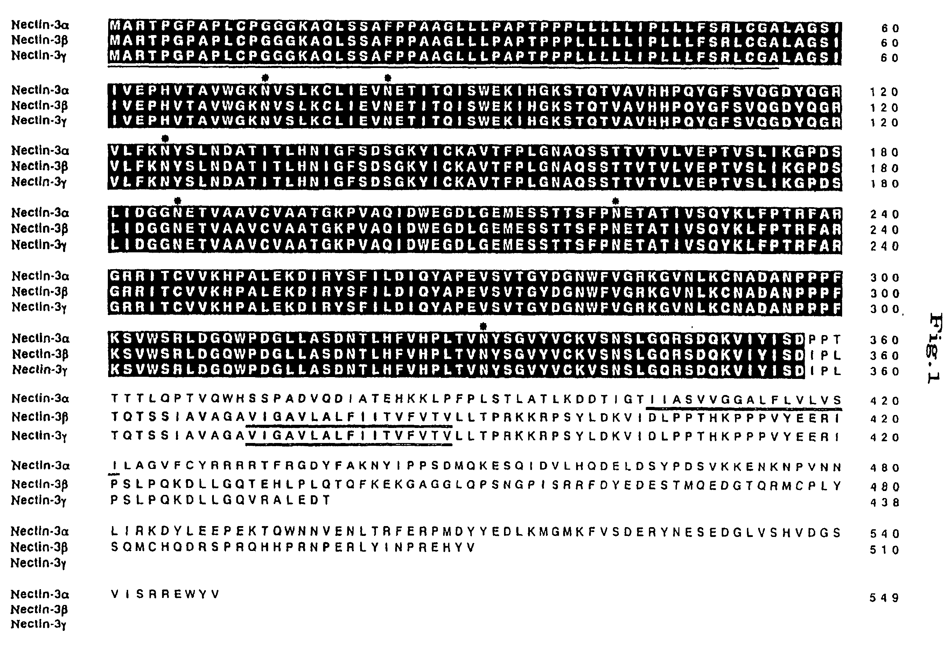

Fig. 1 is to compare the amino acid sequences of nectin-3α, -3β and -3γ. The black background indicates the identical sequence among the three variants; the gray background indicates the identical sequence between nectin-3β and -3γ; the underline indicates the signal peptide; the double underlines indicate the transmembrane regions; and the asterisks indicate the glycosylated sites in asparagine.

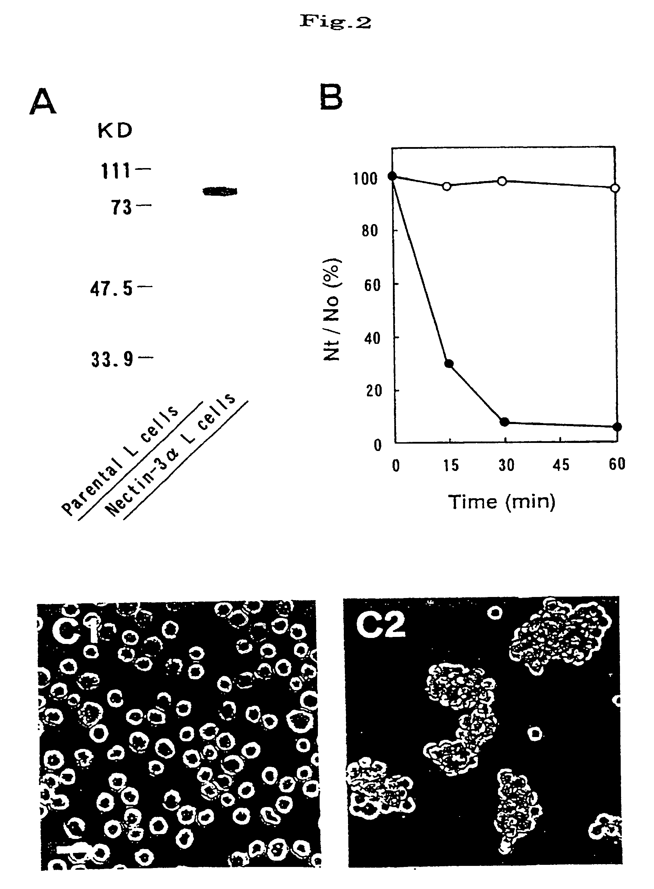

Fig. 2 shows the experimental data of trans homo-interaction of nectin-3α. (A) indicates the expression level of nectin-3α, for which a cell lysate was subjected to development in SDS-PAGE followed by Western blot analysis with a polyclonal anti-nectin-3α antibody. (B) indicates cell aggregation activities of parental L cells (o) and nectin-3α-expressing L cells (•). (C) indicates cell aggregation activities of parental L cells (C1) and nectin-3α-expressing L cells (C2). The bar is 100 µm.

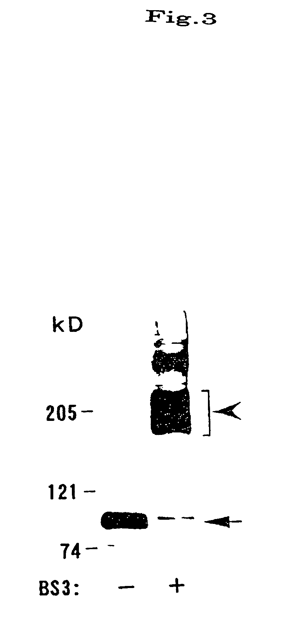

Fig. 3 shows the experimental data of cis homo-dimerization of nectin-3α. Nectin-3α-expressing cells were incubated in the presence or absence of BS3; each cell lysate was subjected to development in SDS-PAGE followed by Western blot analysis with a polyclonal anti-nectin-3α antibody. The arrow indicates the monomer; and the arrowhead indicates the dimer.

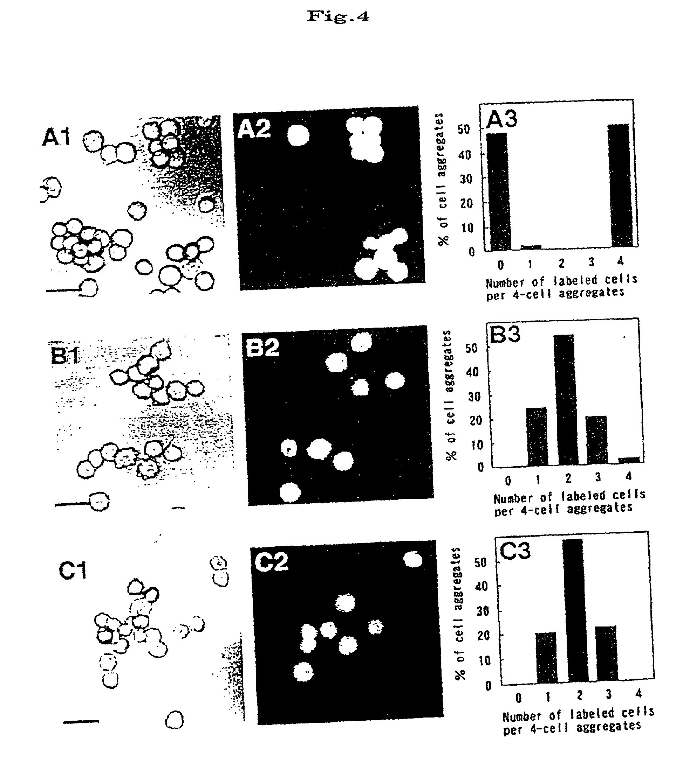

Fig. 4 shows the experimental data of mixed-cell aggregation activities of labeled nectin-1α-expressing L cells and unlabeled nectin-2α-expressing L cells (A1 to A3), labeled nectin-3α-expressing L cells and unlabeled nectin-1α-expressing L cells (B1 to B3), and labeled nectin-3α-expressing L cells and unlabeled nectin-2α-expressing L cells (C1 to C3). A1, B1 and C1 are the images in interference contrast microscopy; A2, B2 and C2 are the images in fluorescence microscopy; A3, B3 and C3 are the data in statistical analysis. The bars are 40 µm.

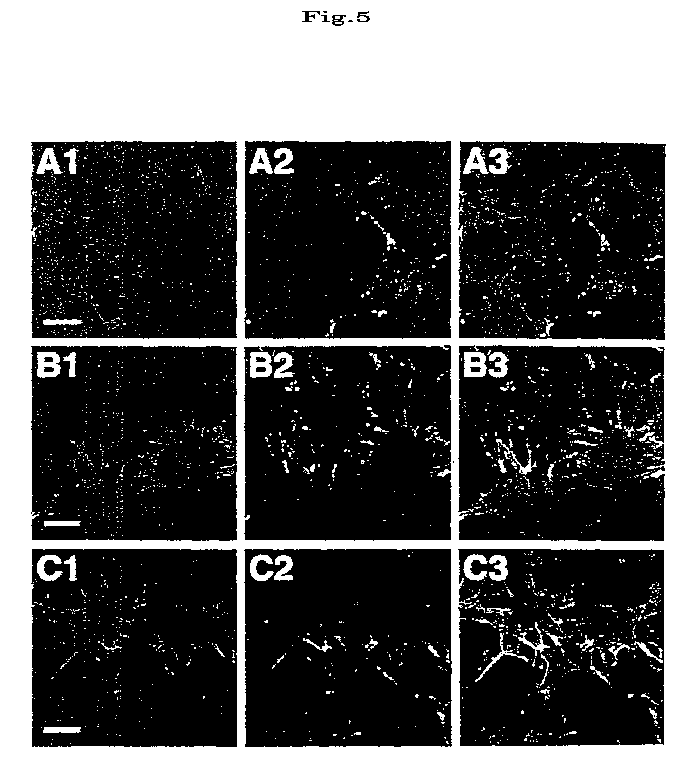

Fig. 5 shows the images in immunofluorescence microscopy of co-cultured two L cell lines of nectin-1α-expressing L cells and -2α-expressing L cells (A1 to A3), nectin-3α-expressing L cells and -1α-expressing L cells (B1 to B3), and nectin-3α-expressing L cells and -2α-expressing L cells (C1 to C3). In A1 and B2, seen is nectin-1α; in A2 and C2, nectin-2α; in B1, nectin-3α (monoclonal antibody); in C1, nectin-3α (polyclonal antibody); and in A3, B3 and C3, merged. The bars are 10 µm.

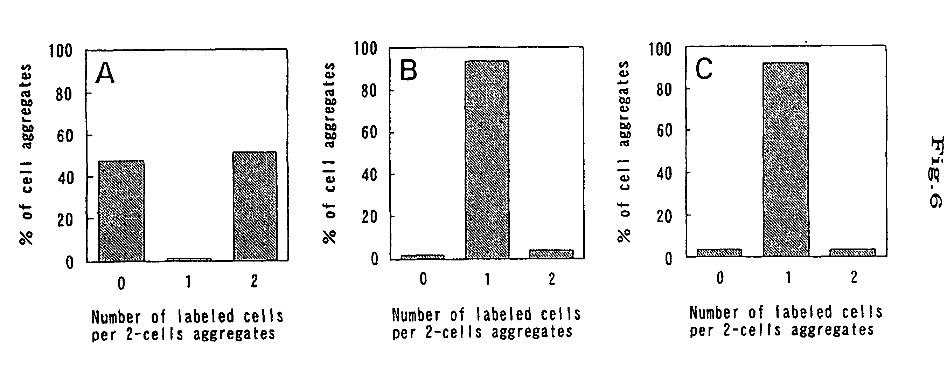

Fig. 6 shows the experimental data of trans hetero-interaction affinity, for which a cell composition (labeled and unlabeled cells) of two-cell aggregates was quantitatively analyzed. A indicates the data of labeled nectin-1α-expressing L cells and unlabeled nectin-2α-expressing L cells; B indicates the data of labeled nectin-3α-expressing L cells and unlabeled nectin-1α-expressing L cells; and C indicates the data of labeled nectin-3α-expressing L cells and unlabeled nectin-2α-expressing L cells.

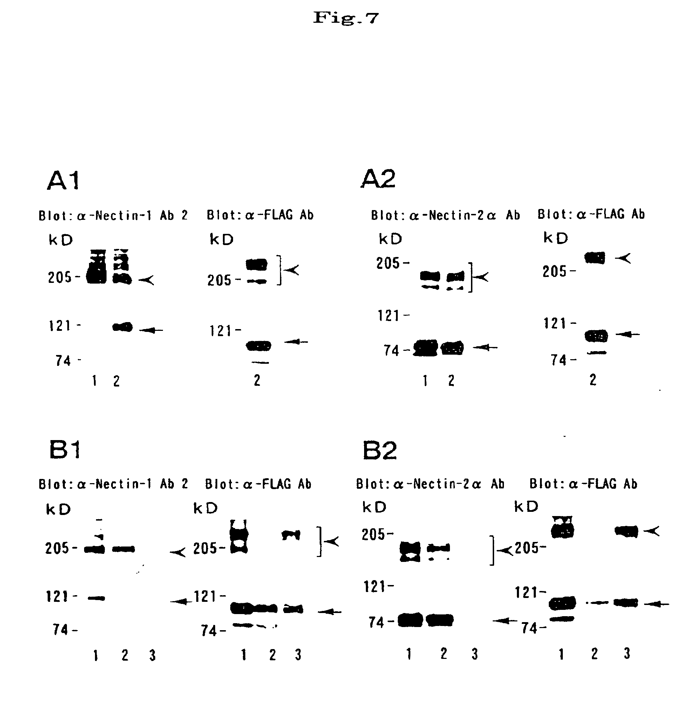

Fig. 7 shows the experimental data of cis hetero-dimerization nectin-3α with nectin-1α or -2α. A is for comparison of cis dimerization. A1 indicates the data of nectin-1α-expressing L cells (lane 1) and -1α/3α-expressing L cells (lane 2); A2 indicates the data of nectin-2α-expressing L cells (lane 1) and -2α/3α-expressing L cells (lane 2). B shows the data of immunoprecipitation. B1 indicates the data of nectin-1α/3α-expressing L cells; B2 indicates the data of nectin-2α/3α-expressing L cells. The cell extract is in lane 1; the supernatant is in lane 2; the precipitate is in lane 3. The arrows indicate monomers; and the arrowheads indicate the dimers.

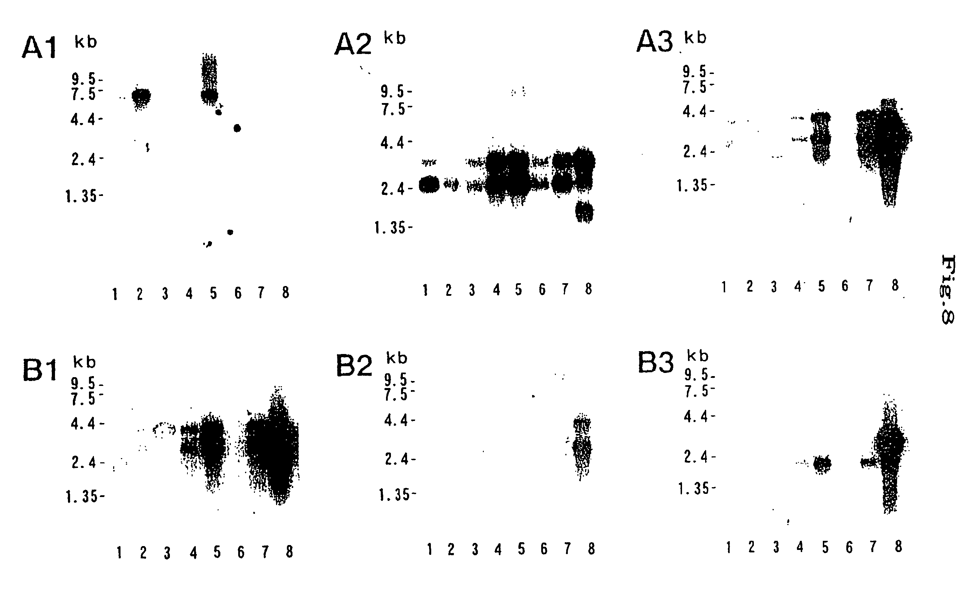

Fig. 8 shows the experimental data of nectin-3 distribution in tissues. A shows the data of Northern blot analysis of nectin-1 (A1), nectin-2 (A2) and nectin-3 (A3). B shows the data of Northern blot analysis of nectin-3α (B1), nectin-3β (B2) and nectin-3γ (B3). Lane 1 is in heart; lane 2 is in brain; lane 3 is in spleen; lane 4 is in lung, lane 5 is in liver, lane 6 is in skeletal muscle; lane 7 is in kidney; and lane 8 is in testis.

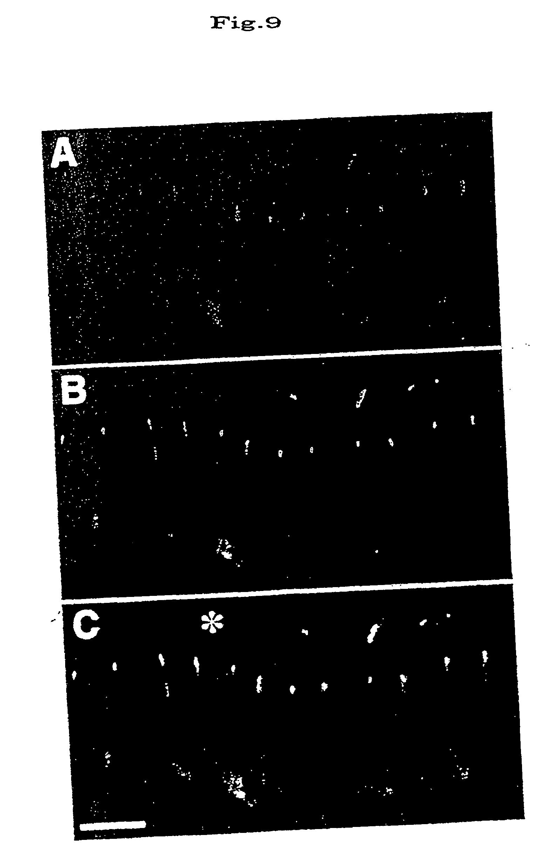

Fig. 9 shows the experimental data of intracellular localization of nectin-3α in mouse small intestine absorptive epithelia. In A, seen is nectin-3α; in B, nectin-2; and in C, merged. The asterisks indicate the inner space of small intestines. The bar is 10 µm.

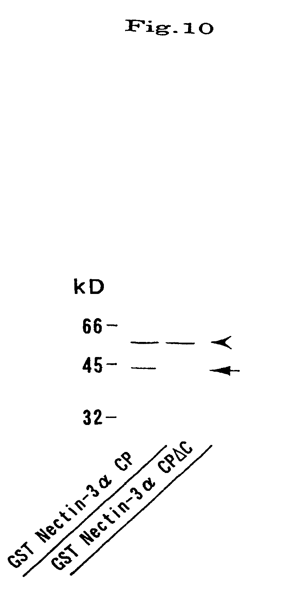

Fig. 10 shows the experimental data of direct binding of afadin to nectin-3α. The arrow indicates a fusion protein, GST-nectin-3α-CP; and the arrowhead indicates a fusion protein, MBP-afadin-PDZ.

### BEST MODE FOR CARRYING OUT THE INVENTION

A mouse protein, nectin-3α is encoded by 1650 bp cDNA having the base sequence of SEQ ID NO: 1, and this has the sequence of 549 amino acids as in SEQ ID NO: 2. The complete cDNA of the nectin-3α has the base sequence of 2178 bp as in SEQ ID NO: 7.

Neetin-3β is a protein encoded by 1533 bp cDNA having the base sequence of SEQ ID NO: 3, and this has the sequence of 510 amino acids as in SEQ ID NO: 4.

Nectin-3γ is a protein encoded by 1317 bp cDNA having the base sequence of SEQ ID NO: 5, and this has the sequence of 435 amino acids as in SEQ ID NO: 6.

These proteins nectin-3 can be obtained in any known method, for example, according to a method of isolating them from mouse and other mammal tissues; according to a method that comprises preparing peptides through chemical synthesis based on the amino acid sequences provided by this invention; or according to a method of recombinant DNA technology using the polynucleotides provided by this invention. Concretely, nectin-3 may be obtained through recombinant DNA technology as follows: An RNA is prepared through in-vitro transcription from a vector having a polynucleotide that encodes nectin-3, and the nectin-3 is expressed in vitro through in-vitro translation using the RNA as a template. In the case of the polynucleotide encoding the nectin-3 being recombined with a suitable expression vector in a known manner, the nectin-3 encoded by the polynucleotide can be abundantly expressed in E. coli, B. subtilis, yeast, animal or plant cells, etc.

The protein nectin-3 of this invention can be expressed in microorganisms such as E. coli, in the following manner The polynucleotide of this invention that encodes the protein is inserted into an expression vector having an origin rcplicavable in microorganisms, a promoter, a ribosome-binding site, a DNA cloning site and a terminator to construct a recombinant expression vector, then host cells are transformed with the expression vector; and the resulting transformant cells are cultured. In that manner, the nectin-3 encoded by the polynucleotide can be abundantly produced in microorganisms. Alternatively, it may be expressed in the form of a fusion protein with any other protein segment. The resulting fusion protein is cleaved with a suitable protease, and only the protein segment encoded by the polynucleotide can be obtained.

The protein nectin-3 of this invention can be expressed in animal cells in the following manner: The polynucleotide of this invention that encodes the protein is recombined with an expression vector for animal cells having a promoter, a splicing region and a poly(A)-addition site, and the recombinant vector is introduced into animal cells, whereby the protein nectin-3 of this invention can be expressed in the thus-transformed animal cells.

The mouse protein nectin-3 obtained according to the method as above can be used, for example, as an antigen to form an antibody that specifically recognizes this protein.

Peptide fragments (of at least 5 amino acid residues) having any partial amino acid sequence of the amino acid sequence of SEQ ID NO: 2, 4 or 6, are also usable as an antigen for constructing the antibody.

The polynucleotide of this invention is a genomic gene of mammals that encodes any of the above-mentioned protein nectin-3α, β or γ. For example, it can be isolated from known genomic libraries, using the polynucleotide having the base sequence of any of SEQ ID NO: 1, 3 or 5 or its partial sequence as a probe.

A polynucleotide of this invention may be cDNA characterized by having the base sequence of SEQ ID NO: 1, 3 or 5, and it encodes any of the above-mentioned nectin-3α, β or γ. Clones of the polynucleotides of this invention can be obtained with ease. Concretely, using an oligonucleotide probe synthesized on the basis of the base sequence of SEQ ID NO: 1, 3 or 5, mouse and other mammal cDNA libraries are screened. Alternatively, using the oligonucleotides as primers, the intended polynucleotides can be synthesized through polymerase chain reaction (PCR).

The antibody of this invention can be obtained as a polyclonal antibody or a monoclonal antibody in any known method of using the protein nectin-3 itself or its partial peptide as an antigen.

The following Examples are to show the experimental data to confirm the structure and the function of the nectin-3 of this invention.

### EXAMPLES

### 1. Procedures

### 1.1 Molecular Cloning of Mouse Nectin-3 cDNAs

From the EST database, three different types of mouse EST clones (AI1428160, AA492633, AA497887), which are similar to but are not the same as nectin-1 and -2, were amplified from a mouse brain cDNA (Clontech). Using the mixture of these cDNAs as a probe, a mouse cDNA library (Stratagene) was screened to obtain a full-length cDNA. For its DNA sequencing, used was a DNA sequencer (ABI 373).

### 1.2 Construction of Nectin-3 Expression Vectors

Vectors pCAGIPuro (J. Biol. Chem., 275: 613-618, 2000), pCAGIPuro-FLAG, p-GEX-KG (Anal. Biochem., 192: 262-267, 1991), pMal-C2 (New England Biolabs Inc.) and pFastBAcl-Msp-Fc (J. Cell. Biol., 145: 539-549, 1999) were used for constructing nectin-3 expression vectors mentioned below. The pCAGIPuro-FLAG was constructed by subcloning a prepro-trypsin signal peptide and the FLAG epitope of p FLAG-CMV1 (Eastman Kodak) into pCAGIPuro.
(a) pCAGIPuro-nectin-3α: 1-549 amino acids (full length) of SEQ ID NO: 2,
(b) pCAGIPuro-FLAG-nectin-3α: 56-549 amino acids of SEQ ID NO: 2,
(c) GST-nectin-3α-CP: 433-549 amino acids of SEQ ID NO: 2 (cytoplasmic region),
(d) GST-nectin-3α-CP-ΔC: 433-545 amino acids of SEQ ID NO: 2 (deletion of the C-terminal four amino acid residues),
(e) GST-nectin-3γ^CP: 397-438 amino acids of SEQ ID NO: 6 (cytoplasmic region),
(f) pFastBac1-Msp-Fc-nectin-3α-EX: 56-400 amino acids of SEQ ID NO: 2 (extracellular region)

### 1.3 Construction of Transformant Cells

L cells (obtained from the Kyoto University) were cultured in a 10 % fetal calf serum-containing DMEM medium to prepare parental L cells for transformation. Full-length human nectin-1α-expressing L cells (nectin-1α-L cells) and full-length mouse nectin-2α-expressing L cells (nectin-2α-L cells) were constructed according to the method described in references (J. Cell Biol., 145: 539-549, 1999; J. Biol. Chem., 275: 613-618, 2000). Full-length mouse nectin-3α-expressing L cells (nectin-3α-L cells) were constructed, using a recombinant vector pCAGIPuro-nectin-3α. Nectin-1α and FLAG-nectin-3α-coexpressing L cells (nectin-1α/3α-L cells), and nectin-2α and FLAG-nectin-3α-coexpressing L cells (nectin-2α/3α-L cells) were constructed by transfecting the nectin-1α-L cells and the nectin-2α-L cells, respectively, with pCAGIPuro-FLAG-nectin-3α, using a lipefectamine reagent (GIBCO BRL). Each cell line was cultured for 1 day, replated, and selected by culturing in the presence of 5 µg/ml of puromycin (Sigma Chemical Co.).

### 1.4 Preparation of Antibodies:

A rabbit antiserum (polyclonal antibody) against nectin-3α was raised against an antigen, GST-nectin-3α-CP. A rat monoclonal antibody against nectin-3 was raised against an antigen, fusion protein of the extracellular region of nectin-3α with IgG Fc. A rabbit polyclonal anti-nectin-1α antibody was prepared according to the method described in a reference (J. Cell Biol., 145: 539-549, 1999). Another rabbit polyclonal anti-nectin-1α antibody was raised against an antigen, synthetic peptide corresponding to the amino acid sequence 450-468 of nectin-1α, according to the method described in the reference (J. Cell Biol., 145: 539-549, 1999). A rat monoclonal anti-nectin-2 antibody was prepared according to the method described in references (J. Cell Biol., 145: 539-549, 1999; Exp. Cell Res., 235: 374-384, 1997); and mouse monoclonal and rabbit polyclonal anti-1-afadin antibodies were prepared according to the method described in references (J. Cell Biol., 139: 517-528, 1997; Oncogene 18: 1609-1618, 1999). A rat monoclonal anti-E-cadherin antibody was obtained from Dr. Takeichi (Kyoto University). A mouse monoclonal anti-FLAG antibody was bought from Eastman Kodak.

### 1.5 Other Procedures

Cell aggregation assay was done according to the method described in references (J. Cell Biol., 145: 539-549, 1999; J. Biol. Chem., 275; 613-618, 2000). For mixed-cell aggregation assay between two different L cell lines, one L cell line was prelabeled with DiI (Molecular Probe Inc., USA), as described (J. Cell Biol., 103: 171-187, 1986).

Chemical cross-linking was done according to the method described in references (Blood 92: 4602-4611, 1998; J. Biol. Chem., 275: 613-618, 2000). Immunoprecipitation was performed according to the method described in references (J. Cell Biol., 145: 539-549, 1999; J. Biol. Chem., 275: 613-618, 2000). Immunofluorescence microscopy of cultured cells was done according to the method described in references (J. Cell Biol., 139: 517-528, J. Cell Biol., 145: 539-549, 1999; J. BioL Chem., 275: 613-618, 2000). Protein concentrations were determined with bovine serum albumin as a control, according to the method described in a reference (Anal. Biochem., 72: 248-254, 1976). SDS-PAGE was done as described (Nature, 227: 680-685, 1970).

Affinity chromatography was performed as follows: The MBP-fusion protein of the PDZ domain of afadin was immobilized on amylose resin beads (New England Biolabs Inc.). GST-nectin-3α-CP and GST-nectin-3α-CP-ΔC were separately applied to the affinity beads. After extensively washed with PBS (containing 0.1 % Triton X-100), the beads were subjected to elution with PBS (containing 20 mM maltose, 0.1 % Triton X-100).

### 2. Results

### 2.1 Cloning and Characterization of Nectin-3 cDNAs

The cDNA clone obtained from the mouse cDNA library has the base sequence of SEQ ID NO: 7, and encoded a protein (calculated molecular weight 60,580) comprising 549 amino acids (SEQ ID NO: 2) in the 1647 bp coding region (SEQ ID NO: 1). This clone contained all the above-mentioned EST clones. The protein was named nectin-3α.

The amino acid sequence of The nectin-3α had an N-terminal hydrophobic signal peptide (1-55 amino acids of SEQ ID NO: 2) and a transmembrane region (405-421 amino acids of SEQ ID NO: 2). The sites for N-linked glycosylation were detected at 73, 83, 125, 186, 222 and 331 amino acids. The nectin-3 contained three Ig-like domains in the extracellular region and a C-terminal conserved motif in the cytoplasmic region (Table 1).

**Table 1 C-Terminal Sequences of the Nectin Family**

| Members | |
|---|---|
| Nectin-1α | SFISKKEWYV |
| Nectin-1β | VRTTEPRGEC |
| Nectin-2α | SLISRRAVYV |
| Nectin-2δ | DEFVSRAMYV |
| Nectin-3α | SVISRREWYV |
| Nectin-3β | LYINPREHYV |
| Nectin-3γ | LGQVRALEDT |

As in the above, these structural properties of nectin-3α are similar to those of nectin-1α, -1β, -2α and -2δ. The extent of homology varied with the regions of the molecules, but the amino acid sequence of the extracellular region of nectin-3α showed 35.9 % and 30.7 % identities to those of nectin-1 and nectin-2, respectively.

During the isolation of nectin-3α, two splicing variants (nectin-3β and -3γ) were isolated. The nectin-3β cDNA (coding region) has a base sequence of 1533 bps (SEQ ID NO: 3), and encodes a protein having an amino acid sequence of 510 amino acids (SEQ ID NO: 4, calculated molecular weight: 55,808). The extracellular region of nectin-3β (1-357 amino acids of SEQ ID NO: 4) is identical to that of nectin-3α, but its transmembrane and cytoplasmic regions (358-510 amino acids of SEQ ID NO: 4) are different from those of nectin-3α. However, nectin-3β also has a C-terminal conserved motif (Table 1).

The nectin-3γ cDNA (coding region) has a base sequence of 1317 bps (SEQ ID NO: 5), and encodes a protein having an amino acid sequence of 438 amino acids (SEQ ID NO: 6, calculated molecular weight: 47,259). The extracellular region, transmembrane region and cytoplasmic region of nectin-3γ are identical to those of nectin-3β, but nectin-3γ lacks the C-terminal conserved motif (Table 1).

Fig. 1 is to compare the amino acid sequences (in one-letter coding) of these nectin-3α, -3β and -3γ.

The following experiments are principally directed to nectin-3α. This is because the data in Northern blot analysis of various tissues confirm that nectin-3α is a major splicing variant (see Fig. 8, B1 to B3).

### 2.2 trans Homo-interaction and cis Homo-dimer Formation

Studies with nectin-1α-L cells and nectin-2α-L cells confirm that nectin-1α and nectin-2α show cell-cell adhesion activity (trans homo-interaction) (J. Cell Biol., 145: 539-549, 1999; J. Biol. Chem., 275: 613-618, 2000). With that, here in examined was whether nectin-3α also shows the same activity. The polyclonal anti-nectin-3α antibody recognized two protein bands with molecular masses of about 100 kDa in the expression products by nectin-3α-L cells (Fig. 2A, Fig. 3). This may be due to the different levels of the post-translational modification such as glycosylation. These molecular masses are different from the calculated molecular weight based on the deduced amino acid sequence. This difference may also be due to the glycosylation. The expression level of nectin-1α, -2α and -3α in each cell line was almost the same (data not shown).

Nectin-3α-L cells were tested for the cell aggregation activity of nectin-3α. As a result, the cell aggregation activity of nectin-3α varied time-dependently (Fig. 2B, C1, C2). EDTA added to nectin-3α did not have any influence on this activity of nectin-3α (data not shown). This confirms that the cell-cell adhesion activity of nectin-3α does not depend on Ca²⁺. These results indicate that nectin-3α is a Ca²⁺-independent homophilic CAM (cell adhesion molecule), like nectin-1α, -2α and -2δ (J. Cell Biol., 145: 539-549, 1999; Exp. Cell Res., 235: 374-384, 1997; Blood, 92: 4602-4611, 1998; J. Biol. Chem., 275: 613-618, 2000).

Nectin-1α and -2α are known to show cis homo-dimerization (J. Biol. Chem., 275: 613-618, 2000). With that, nectin-3α was also tested for the same activity. Nectin-3α-L cells were dissociated to a single-cell suspension and incubated with a cell surface cross-iinker, BS3, and then subjected to Western blot analysis using a polyclonal anti-nectin-3α antibody. The cross-linking of the cell line resulted in the formation of additional bands with molecular masses of about 200 to 220 kDa that correspond to dimers (Fig. 3). Bands with higher molecular masses were also detected. Because the cross-linking was done in a single-cell suspension, it is most likely that the dimers and oligomers are derived from the cis homo-interaction rather than from the trans homo-interaction.

### 2.3 trans Hetero-interaction of Nectin-3α with Nectin-1α or -2α

To examine whether each member of the nectin family shows heterophilic cell-cell adhesion activity (trans hetero-interaction), mixed-cell aggregation assay was performed. When DiI-labeled nectin-1α-L cells were mixed with unlabeled nectin-2α-L cells, the resulting aggregates exclusively consisted of the labeled cells alone or the unlabeled cells alone (Fig. 4 A1-A3). Few aggregates consisting of both the labeled and unlabeled cells were detected. In contrast, when DiI-labeled nectin-3α-L cells were mixed with unlabeled nectin-1α-L cells, the resulting aggregates consisted of both the labeled cells and the unlabeled cells (Fig. 4, B1-B3). This indicates that nectin-3α shows trans hetero-interaction with nectin-1α. The similar result was obtained with nectin-3α and nectin-2α (Fig. 4, C1-C3).

To confirm these results, immunofluorescence microscopy was performed. When nectin-1α-L cells were co-cultured with nectin-2α-L cells, nectin-1α and nectin-2α were localized in the cell-cell contact sites of the respective L cells (Fig. 5, A1-A3). However, neither nectin-1α nor nectin-2α was detected in the contact sites of the two types of the L cells. In contrast, when nectin-3α-L cells were co-cultured with nectin-1α-L cells, nectin-3α and nectin-1α coexisted in the cell-cell contact site of the two types of the L cells (Fig. 5, B1-B3). The same result was obtained in co-culture of nectin-3α-L cells and nectin-2α-L cells (Fig. 5, C1-C3). These result indicate that nectin-3α shows trans hetero-interaction with nectin-1α and -2α, whereas nectin-1α does not trans hetero-interaction with nectin-2α.

To determine whether each member of the nectin family prefers trans homo-interaction or trans hetero-interaction, two-cell aggregates were analyzed through mixed-cell aggregation assay. Consistent with the result in the analysis of two-cell aggregates, the mixture of nectin-1α-L cells and nectin-2α-L cells resulted in the formation of homotypic two-cell aggregates (Fig. 6A). In contrast, the mixture of nectin-1α-L cells and nectin-3α-L cells resulted in the formation of heterotypic two-cell aggregates, and few homotypic two-cell aggregates were found (Fig. 6B). The similar result was obtained when nectin-3α-L cells and nectin-2α-L cells were mixed (Fig. 6C). These results indicate that the affinity of trans hetero-interaction of nectin-3α with nectin-1α or -2α is obviously higher than that of trans homo-interaction of nectin-1α, -2α or -3α.

### 2.4 cis Hetero-dimerization of Nectin-3α with Nectin-1α or -2α

Next examined was whether nectin-3α forms a cis hetero-dimer with nectin-1α or -2α. Using nectin-1α/3α-L cells and nectin-2α/3α-L cells, FLAG-nectin-3α was expressed in the nectin-1α-L cells and the nectin-2α-L cells. As a result, the FLAG-nectin-3α expressed did not change the size of the cis dimers of nectin-1α or -2α (Fig. 7, A1 and A2). When nectin-1α/3α-L cells were subjected to cell surface cross-linking, followed by immunoprecipitation using the monoclonal anti-FLAG antibody, then nectin-1α was recovered in the supernatant and was not coimmunoprecipitated with FLAG-nectin-3α (Fig. 7, B1). The similar result was obtained with nectin-2α/3α-L cells (Fig. 7, B2). These results indicate that nectin-3α does not form a cis hetero-dimer with nectin-1α or -2α.

### 2.5 Tissue Distribution and Intracellular Localization of Nectin-3α

Consistent with previous reports (J. Virol., 66: 2807-2813, 1992; Gene 155: 261-265,1995; Gene 159: 267-272, 1995), Northern blot analysis revealed that nectin-1 is abundantly expressed in brain whereas nectin-2 is ubiquitously expressed (Fig. 8, A1 and A2). Northern blot analysis using, as a probe, the coding region common to the three splicing variants of nectin-3 revealed some mRNA bands in various tissues (Fig. 8, A3). To determine the tissue distribution of each splicing variant, a cDNA probe specific to each variant was used. Nectin-3α gave about 5.2-kb, 3.8-kb, 3.3-kb and 2.7-kb mRNA bands, abundantly expressed in testis but slightly in other tissues (heart, brain, lung, liver and kidney) (Fig. 8, B1). Nectin-3β gave about 5.2-kb and 3.3-kb mRNA bands, expressed in testis (Fig. 8, B2). Nectin-3γ gave an about 3.3-kb mRNA band in testis, and an about 2.1-kb mRNA band in lung, liver and kidney (Fig. 8, B3).

To determine the intracellular localization of nectin-3α, immunofluorescence microscopy was performed, using a polyclonal anti-nectin-3α antibody. Nectin-3α was colocalized with nectin-2 in the junctional complex regions in mouse small intestine absorptive epithelia (Fig. 9). These results suggest that nectin-3α is also localized at cadherin-based cell-cell AJs, like nectin-2 (J. Cell Biol., 145: 539-549, 1999).

### 2.6 Direct Binding of Nectin-3 to Afadin

To confirm whether nectin-3α directly binds to afadin, affinity chromatography was performed. The GST-fusion protein in the cytoplasmic region of nectin-3α (GST-nectin-3α-CP) bound to the MBP-fusion protein in the PDZ domain of afadin (MBP-afadin-PDZ) immobilized on amylose resin beads (Fig. 10). The stoichiometry of binding of nectin-3α to afadin was about 1:1. In contrast, the GST-fusion protein in the C-terminal four amino acid residues-deleted cytoplasmic region of nectin-3α (GST-nectin-3α-CP-ΔC) did not bind to the affinity beads. Similarly, the GST-fusion protein in the cytoplasmic region of nectin-3γ that lacks the C-terminal conserved motif did not also bind to the affinity beads.

### INDUSTRIAL APPLICABILITY

As described in detail hereinabove, the invention of this application provides a novel protein nectin-3 that belongs to one and the same protein family to which nectin-1 and -2 belong. The protein provides important information for clarifying all aspects of the molecular mechanism in cell-cell binding systems, and, in addition, it leads to the possibility of clarifying the mechanism of, for example, humectation and metastasis of carcinoma, and is expected to be applicable to diagnosis of carcinoma for its malignancy and to a method for treating cases with carcinoma and also to development of medicines for carcinoma.

## Claims

1. A protein nectin-3 consisting of the amino acid sequence of SEQ ID NO: 2.

2. A protein nectin-3 consisting of the amino acid sequence of SEQ ID NO: 4.

3. A protein nectin-3 consisting of the amino acid sequence of SEQ ID NO: 6.

4. A polynucleotide encoding the protein nectin-3 of any of claims 1 to 3.

5. The polynucleotide as claimed in claim 4, which has the base sequence of SEQ ID NO: 1.

6. The polynucleotide as claimed in claim 4, which has the base sequence of SEQ ID NO: 3.

7. The polynucleotide as claimed in claim 4, which has the base sequence of SEQ ID NO: 5.

8. A recombinant vector having the polynucleotide of any of claims 4 to 7.

9. An antibody against the protein nectin-3 of any of claims 1 to 3.

## Patentansprüche

1. Nectin-3-Protein, bestehend aus der Aminosäuresequenz SEQ ID NO:2.

2. Nectin-3-Protein, bestehend aus der Aminosäuresequenz SEQ ID NO:4.

3. Nectin-3-Protein, bestehend aus der Aminosäuresequenz SEQ ID NO:6.

4. Polynukleotid, welches das Nectin-3-Protein nach einem der Ansprüche 1 bis 3 kodiert.

5. Polynukleotid nach Anspruch 4, das die Basensequenz SEQ ID NO:1 aufweist.

6. Polynukleotid nach Anspruch 4, das die Basensequenz SEQ ID NO:3 aufweist.

7. Polynukleotid nach Anspruch 4, das die Basensequenz SEQ ID NO:5 aufweist.

8. Rekombinanter Vektor, der das Polynukleotid nach einem der Ansprüche 4 bis 7 aufweist.

9. Antikörper gegen das Nektin-3-Protein nach einem der Ansprüche 1 bis 3.

## Revendications

1. Protéine nectine-3 constituée par la séquence d'acides aminés de SEQ ID n° 2.

2. Protéine nectine-3 constituée par la séquence d'acides aminés de SEQ ID n° 4.

3. Protéine nectine-3 constituée par la séquence d'acides aminés de SEQ ID n° 6.

4. Polynucléotide codant la protéine nectine-3 selon l'une quelconque des revendications 1 à 3.

5. Polynucléotide selon la revendication 4, qui a la séquence de bases de SEQ ID n° 1.

6. Polynucléotide selon la revendication 4, qui a la séquence de bases de SEQ ID n° 3.

7. Polynucléotide selon la revendication 4, qui a la séquence de bases de SEQ ID n° 5.

8. Vecteur recombinant ayant le polynucléotide selon l'une quelconque des revendications 4 à 7.

9. Anticorps contre la protéine nectine-3 selon l'une quelconque des revendications 1 à 3.
